(19) **Europäisches Patentamt / European Patent Office / Office européen des brevets**

(11) **EP 1 963 505 B1**

(12) **EUROPEAN PATENT SPECIFICATION**

(45) Date of publication and mention
of the grant of the patent:
**22.08.2012 Bulletin 2012/34**

(51) Int Cl.:
*C12N 9/88* (2006.01)     *C12N 15/10* (2006.01)
*C12N 15/82* (2006.01)     *C12Q 1/68* (2006.01)

(21) Application number: **06835671.6**

(22) Date of filing: **20.12.2006**

(86) International application number:
**PCT/NL2006/000649**

(87) International publication number:
**WO 2007/073166 (28.06.2007 Gazette 2007/26)**

(54) **IMPROVED TARGETED NUCLEOTIDE EXCHANGE WITH PROPYNYL MODIFIED OLIGONUCLEOTIDES**

VERBESSERTER GEZIELTER NUKLEOTIDAUSTAUSCH MIT PROPINYL-MODIFIZIERTEN OLIGONUKLEOTIDEN

ECHANGE AMELIORE CIBLE DE NUCLEOTIDES AVEC DES OLIGONUCLEOTIDES MODIFIES PAR PROPYNYLE

(84) Designated Contracting States:
**AT BE BG CH CY CZ DE DK EE ES FI FR GB GR HU IE IS IT LI LT LU LV MC NL PL PT RO SE SI SK TR**

(30) Priority: **22.12.2005 PCT/NL2005/000884**
**09.05.2006 PCT/NL2006/000244**

(43) Date of publication of application:
**03.09.2008 Bulletin 2008/36**

(73) Proprietor: **KEYGENE N.V.**
**6700 AE Wageningen (NL)**

(72) Inventors:
• **BUNDOCK, Paul**
**1391 XW ABCOUDE (NL)**
• **DE BOTH, Michiel, Theodoor, Jan**
**NL-6708 BG Wageningen (NL)**
• **HOGERS, René, Cornelis, Josephus**
**NL-6715 GR EDE (NL)**

(74) Representative: **de Lang, Robbert-Jan et al**
**Exter Polak & Charlouis B.V. (EP&C)**
**J.J. Viottastraat 31**
**1071 JP Amsterdam (NL)**

(56) References cited:
EP-A- 1 152 058     WO-A-01/73002
WO-A-01/92512     WO-A-02/26967
WO-A2-00/24885

• HE JUNLIN ET AL: "Propynyl groups in duplex DNA: stability of base pairs incorporating 7-substituted 8-aza-7-deazapurines or 5-substituted pyrimidines." NUCLEIC ACIDS RESEARCH 15 DEC 2002, vol. 30, no. 24, 15 December 2002 (2002-12-15), pages 5485-5496, XP002424169 ISSN: 1362-4962 cited in the application
• FROEHLER B C ET AL: "OLIGODEOXYNUCLEOTIDES CONTAINING C-5 PROPYNE ANALOGS OF 2'-DEOXYURIDINE AND 2'-DEOXYCYTIDINE" TETRAHEDRON LETTERS, ELSEVIER, AMSTERDAM, NL, vol. 33, no. 37, January 1992 (1992-01), pages 5307-5310, XP002018815 ISSN: 0040-4039
• PAREKH-OLMEDO HETAL ET AL: "Targeted nucleotide exchange in Saccharomyces cerevisiae directed by short oligonucleotides containing locked nucleic acids." CHEMISTRY AND BIOLOGY (CAMBRIDGE), vol. 9, no. 10, October 2002 (2002-10), pages 1073-1084, XP002424170 ISSN: 1074-5521
• BUSUTTIL S J ET AL: "Antisense Suppression of Protein Kinase C-Alpha and Delta in Vascular Smooth Muscle", JOURNAL OF SURGICAL RESEARCH, ACADEMIC PRESS INC., SAN DIEGO, CA, US, vol. 63, no. 1, 1 June 1996 (1996-06-01), pages 137-142, XP008081231, ISSN: 0022-4804, DOI: DOI:10.1006/JSRE.1996.0236

**Description**

Field of the invention

The present invention relates to a method for the specific and selective alteration of a nucleotide sequence at a specific site of the DNA in a target cell by the introduction into that cell of an oligonucleotide. The result is the targeted exchange of one or more nucleotides so that the sequence of the target DNA is converted to that of the oligonucleotide where they are different. More in particular, the invention relates to the targeted nucleotide exchange using modified oligonucleotides. The invention further relates to oligonucleotides and kits. The invention also relates to the application of the method.

Background of the invention

Genetic modification is the process of deliberately creating changes in the genetic material of living cells with the purpose of modifying one or more genetically encoded biological properties of that cell, or of the organism of which the cell forms part or into which it can regenerate. These changes can take the form of deletion of parts of the genetic material, addition of exogenous genetic material, or changes in the existing nucleotide sequence of the genetic material. Methods for the genetic modification of eukaryotic organisms have been known for over 20 years, and have found widespread application in plant, human and animal cells and micro-organisms for improvements in the fields of agriculture, human health, food quality and environmental protection. The common methods of genetic modification consist of adding common methods of genetic modification consist of adding exogenous DNA fragments to the genome of a cell, which will then confer a new property to that cell or its organism over and above the properties encoded by already existing genes (including applications in which the expression of existing genes will thereby be suppressed). Although many such examples are effective in obtaining the desired properties, these methods are nevertheless not very precise, because there is no control over the genomic positions in which the exogenous DNA fragments are inserted (and hence over the ultimate levels of expression), and because the desired effect will have to manifest itself over the natural properties encoded by the original and well-balanced genome. On the contrary, methods of genetic modification that will result in the addition, deletion or conversion of nucleotides in predefined genomic loci will allow the precise modification of existing genes.

Oligonucleotide-directed Targeted Nucleotide Exchange (TNE, sometimes ODTNE) is a method, that is based on the delivery into the eukaryotic cell nucleus of synthetic oligonucleotides (molecules consisting of short stretches of nucleotide-like moieties that resemble DNA in their Watson-Crick basepairing properties, but may be chemically different from DNA) (Alexeev and Yoon, Nature Biotechnol. 16: 1343, 1998; Rice, Nature Biotechnol. 19: 321, 2001; Kmiec, J. Clin. Invest. 112: 632, 2003). By deliberately designing a mismatch nucleotide in the homology sequence of the oligonucleotide, the mismatch nucleotide may be incorporated in the genomic DNA sequence. This method allows the conversion of single or at most a few nucleotides in existing loci, but may be applied to create stop codons in existing genes, resulting in a. disruption of their function, or to create codon changes, resulting in genes encoding proteins with altered amino acid composition (protein engineering).

Targeted nucleotide exchange (TNE) has been described in plant, animal and yeast cells. The first TNE reports utilized a so-called chimera that consisted of a self-complementary oligonucleotide that is designed to intercalate at the chromosomal target site. The chimera contains a mismatched nucleotide that forms the template for introducing the mutation at the chromosomal target. In order to select for TNE events, most studies attempt to introduce a single nucleotide change in an endogenous gene that leads to herbicide resistance. The first examples using chimeras came from human cells (see the-review Rice et al. Nat.Biotech. 19: 321-326). The use of chimeras has also been successful in the plant species tobacco, rice, and maize (Beetham et al. 1999 Proc.Natl.Acad.Sci.USA 96: 8774-8778; Kochevenko et al. 2003 Plant Phys. 132: 174-184; Okuzaki et al. 2004 Plant Cell Rep. 22: 509-512). However, the activity of chimeras was found to be difficult to reproduce and so the TNE activity of single stranded (ss) oligonucleotides has been tested. These have been found to give more reproducible results in wheat, yeast and human cells (Liu et al. 2002 Nuc.Acids Res. 30: 2742-2750; review, Parekh-Olmedo et al. 2005 Gene Therapy 12: 639-646; Dong et al. 2006 Plant Cell Rep. 25: 457-65).

Several groups have shown that TNE can also be detected using total cellular protein extracts. Such assays for TNE activity are called cell free assays (Cole-Strauss et al. 1999 Nucleic Acids Res. 27: 1323-1330; Gamper et al. 2000 Nucleic Acids Res. 28, 4332-4339; Kmiec et al. 2001 Plant J. 27: 267-274; Rice *et al.* 2001 40: 857-868). The assay is setup as follows. A plasmid containing two bacterial antibiotic resistance genes (kanamycin and carbenicillin) is mutated so that one of the antibiotic resistance genes (e.g. kanamycin) contains an in frame stop codon due to the alteration of a single nucleotide (e.g. TAT to TAG). This mutated plasmid is then incubated with total cellular protein and a singe stranded oligonucleotide designed to correct the stop codon in the antibiotic resistance gene. The proteins necessary for TNE are present in the cellular extract and utilize the oligonucleotide to alter the stop codon in the antibiotic resistance gene, restoring the resistance phenotype. Plasmid DNA is then purified from the reaction mixture and transformed to E.coli. The bacteria are then plated out on media containing kanamycin and the number of bacterial colonies represents the number of TNE repair events. The electroporation efficiency is calculated by counting the number of colonies growing on media containing carbenicllin. The TNE efficiency can be quantified by calculating the ratio of repaired plasmids against the total number of transformed plasmids.

In such an experiment, the oligonucleotide effects a substitution, altering TAG to TAC. Furthermore, the cell free system can also be used to study the possibility of using oligonucleotides to produce single nucleotide insertions. Plasmids can be produced which have a single nucleotide deleted from the antibiotic resistance gene, generating a frame shift mutation. In the cell free assay, the deletion is repaired by addition of a nucleotide mediated by the oligonucleotide.

The greatest problem facing the application of TNE in cells of higher organisms such as plants is the low efficiency that has been reported so far. In maize Zhu et al. (2000 Nature Biotech. 18: 555-558) reported a conversion frequency of $1 \times 10^{-4}$. Subsequent studies in tobacco (Kochevenko et al. 2003 Plant Phys. 132: 174-184) and rice (Okuzaki et al. 2004 Plant Cell Rep. 22: 509-512) have reported frequencies of $1 \times 10^{-6}$ and $1 \times 10^{-4}$ respectively. These frequencies remain too low for the practical application of TNE.

Faithful replication of DNA is one of the key criteria that mediates maintenance of genome stability and ensures that the genetic information contained in the DNA is passed on free of mutation from one generation to the next. Many errors arise from damage in the parental DNA strand or are generated by agents that react with DNA bases (UV light, environmental toxins). Every organism must maintain a safeguard to prevent or correct these mutations. The mismatch repair system (MMR) is thought to recognize and correct mismatched or unpaired bases caused during DNA replication, in DNA damage surveillance and in prevention of recombination between non-identical sequences (Fedier and Fink, 2004 Int. J Oncol. 2004 ; 24 (4):1039-47), and contributes to the fidelity of DNA replication in living cells.

TNE has been described in a variety of patent applications of Kmiec, *inter alia* in WO0173002, WO03/027265, WO01/87914, WO99/58702, WO97/48714, WO02/10364. In WO 01/73002 it is contemplated that the low.efficiency of gene alteration obtained using unmodified DNA oligonucleotides is largely believed to be the result of degradation of the donor oligonucleotides by nucleases present in the reaction mixture or the target cell. To remedy this problem, it is proposed to incorporate modified nucleotides that render the resulting oligonucleotides resistant against nucleases. Typical examples include nucleotides with phosphorothioate linkages, 2'-O-methyl-analogs or locked nucleic acids (LNAs). These modifications are preferably located at the ends of the oligonucleotide, leaving a central DNA domain surrounding the targeted base. Furthermore the publication stipulates that specific chemical interactions are involved between the converting oligonucleotide and the proteins involved in the conversion. The effect of such chemical interactions to produce nuclease resistant termini using modification other than LNA, phosphorothioate linkages or 2'-O-methyl analogue incorporation in the oligonucleotide is impossible to predict because the proteins involved in the alteration process and their chemical interaction with the oligonucleotide substituents are not yet known and, according to the inventors of WO0173002, cannot be predicted.

As the efficiency of the current methods of ODTNE is relatively low (as stated previously, between $10^{-6}$ and $10^{-4}$, despite reported high delivery rates of the oligonucleotide of 90%) there is a need in the art to come to methods for TNE that are more efficient. Accordingly, the present inventors have set out to improve on the existing TNE technology.

### *Description of the invention*

Embodiments of the present invention are disclosed in the accompagning claims. The present inventors have now found that by incorporating nucleotides into.the donor oligonucleotide for TNE that are capable of binding more strongly to the acceptor DNA than the corresponding unmodified nucleotides like A,C,T, or G, the rate of TNE can be increased significantly. Without being bound by theory, the present inventors believe that by the incorporation of modified nucleotides into the donor oligonucleotide, the donor oligonucleotide binds more strongly to the acceptor DNA and hence increases the ratio of TNE. The present inventors have found that oligonucleotides comprising C5-propyne modified pyrimidines and/or C7 propynyl modified purines improves the efficiency of TNE significantly.

To this end, the effect of using oligonucleotides incorporating propynylated nucleosides on the frequency of TNE in the cell free system has been investigated. The TNE activity of such oligonucleotides was compared with the TNE activity of oligonucleotides made up of normal DNA. It was found that oligonucleotides containing C5-propyne pyrimidines and/or C7 propynylated purines increased the TNE efficiency for both substitutions and insertions in the cell free assay to a level hitherto unobserved. The oligonucleotides containing propynylated nucleotides were up to 10 fold more efficient in the cell free assay compared to oligonucleotides made up of normal DNA. It was also found that the TNE efficiency could be further improved with increasing the number of propynylated nucleotides in the oligonucleotide and furthermore that the improvement observed was locus independent, indicating that oligonucleotides containing C5-propyne pyrimidines and/or C7 propynylated purines are capable of providing enhanced frequencies of TNE in a species independent manner, such as in plant and animal cells.

The present disclosure is thus based on the disclosure consideration that the desired targeted nucleotide exchange can be achieved by the use of (partly) C5 propyne modified pyrimidines and / or C7 propyne modified purines oligonucleotides. The location, type and amount of modification (i.e. status) of the oligonucleotide can be varied as will be disclosed herein below.

The present disclosure thus, in one aspect provides ((fully) C5 propyne modified pyrimidine and or C7 propyne modified purines) oligonucleotides. The modified ss-oligonucleotides can be used to introduce specific genetic changes in plant and animal or human cells. The disclosure is applicable in the field of biomedical research, agriculture and to construct specifically mutated plants and animals, including humans. The disclosure is also applicable in the field of medicine and gene therapy.

The sequence of an oligonucleotide of the disclosure is homologous to the target strand except for the part that contains a mismatch base that introduces the base change in the target strand. The mismatched base is introduced into the target sequence. By manipulating the modification (compared to conventional A, C, T, or G) of the nucleotides, and more in particular, by manipulating the location and amount of propyne modification oligonucleotide that introduces the mismatch, the efficiency (or the degree of successful incorporation of the desired nucleotide at the desired position in the DNA duplex) can be improved.

Another aspect of the disclosure resides in a method for the targeted alteration of a parent DNA strand (first strand, second strand) by contacting the parent DNA duplex with an oligonucleotide that contains at least one mismatch nucleotide compared to the parent strand, wherein the donor oligonucleotide contains a section that is modified with C5-propyne substituted pyrimidines and/or C7 substituted purines to have a higher binding capacity than the parent (acceptor) strand in the presence of proteins that are capable of targeted nucleotide exchange.

Thus, the gist of the disclosure lies in the improvement in the binding capacity, of the intercalating oligonucleotide (sometimes referred to as the donor) with propyne modified ss-oligonucleotides relative to the unmodified intercalating oligonucleotide.

## *Detailed description of the disclosure*

In one aspect, the disclosure pertains to an propyne modified oligonucleotide for targeted alteration of a duplex DNA sequence. The duplex DNA sequence contains a first DNA sequence and a second DNA sequence. The second DNA sequence is the complement of the first DNA sequence and pairs to it to form a duplex. The oligonucleotide comprises a domain that comprises at least one mismatch with respect to the duplex DNA sequence to be altered. Preferably, the domain is the part of the oligonucleotide that is complementary to the first strand, including the at least one mismatch.

Preferably, the mismatch in the domain is with respect to the first DNA sequence. The oligonucleotide comprises a section that is modified (contains C5-propyne modified pyrimidine and/or C7 propyne modified purines) to have a higher binding affinity than the (corresponding part of the) second DNA sequence.

The domain that contains the mismatch and the section containing the modified nucleotide(s) may be overlapping. Thus, in certain embodiments, the domain containing the mismatch is located at a different position on the oligonucleotide than the section of which the modification is considered. In certain embodiments, the domain incorporates the section. In certain embodiments the section can incorporate the domain. In certain embodiments the domain and the section are located at the same position on the oligonucleotide and have the same length i.e. they coincide in length and position. In certain embodiments, there can be more than one section within a domain.

For the present disclosure, this means that the part of the oligonucleotide that contains the mismatch which is to be incorporated in the DNA duplex can be located at a different position from the part of the oligonucleotide that is modified. In particular, in certain embodiments wherein the cell's repair system (or at least the proteins involved with this system, or at least proteins that are involved in TNE) determines which of the strands contain the mismatch and which strand is to be used as the template for the correction of the mismatch.

In certain embodiments, the oligonucleotide comprises a section that contains at least one, preferably at least 2, more preferably at least 3 propyne modified nucleotide(s). In certain embodiments, the section on the oligonucleotide can contain more than 4, 5, 6, 7, 8, 9, or 10 propyne modified nucleotides. In certain embodiments the section is fully modified i.e. all pyrimidines in the oligonucleotide carry a C5-propyne substitution and/or all purines carry a C7-propyne substitution. In certain embodiments, at least 10 % of the nucleotides in the oligonucleotide is replaced by its propynylated counterpart. In certain embodiments at least 25, more preferably at least 50%, even more preferably at least 75% and in some cases it is preferred that at least 90 % of the nucleotides are replaced by their propynylated counterparts.

In certain embodiments, more than one mismatch can be introduced, either simultaneously or successively. The oligonucleotide can accommodate more than one mismatch on either adjacent or removed locations on the oligonucleotide. In certain embodiments the oligonucleotide can comprise two, three, four or more mismatch nucleotides which may be adjacent or remote (i.e. non-adjacent). The oligonucleotide can comprise further domains and sections to accommodate this, and in particular can comprise several sections. In certain embodiments, the oligonucleotide may incorporate a potential insert that is to be inserted in the acceptor strand. Such an insert may vary in length from more than five up to 100 nucleotides. In a similar way in certain embodiments, deletions can be introduced of similar length variations (from 1 to 100 nucleotides).

In a further aspect of the disclosure, the design of the oligonucleotide can be achieved by:

- determining the sequence of the acceptor strand, or at least of a section of the sequence around the nucleotide to be exchanged. This can typically be in the order of at least 10, preferably 15, 20, 25 or 30 nucleotides adjacent to the mismatch, preferably on each side of the mismatch, (for example GGGGGGXGGGGGG, wherein X is the mismatch);
- designing a donor oligonucleotide that is complementary to one or both the sections adjacent to the mismatch and contains the desired nucleotide to be exchanged (for example CCCCCCYCCCCCC);
- providing (e.g. by synthesis) the donor oligonucleotide with propyne modifications at desired positions. Modifications may vary widely, depending on the circumstances. Examples are $C^mC^mC^mC^mC^mC^mC^mYC^mC^mC^mC^mC^mC^m$, $C^m$ $CC^mCC^mCYC^mCC^mCC^mC$, $CCCCCCYC^mC^mC^mC^mC^mC^m$, $C^mC^mC^mC^mC^mC^mC^mYCCCCCC$, $CCCCC^mYC^m$ CCCCC, $C^mCCCCCYC^mCCCCC$, $C^mCCCCCYCCCCCC^m$, $C^mCCCCCYCCCCCC$, and so on, wherein $C^m$ stands for a propyne modified nucleotide residue. For a different acceptor sequence, e.g. ATGCGTACXGTCCATGAT, corresponding donor oligonucleotides can be designed, e.g. TACGCATGYCAGGTACTA with modification as variable as outlined hereinbefore.
- subjecting the DNA to be modified with the donor oligonucleotide in the presence of proteins that are capable of targeted nucleotide exchange, for instance, and in particular, proteins that are functional in the mismatch repair mechanism of the cell.

Without being bound by theory, improved binding affinity is thought to increase the likelihood that an oligonucleotide finds and remains bound to its target, thus improving the TNE efficiency. Many different chemical modifications of the sugar backbone or the base confer and improved binding affinity. The present inventors however, chose to focus on propyne modified oligonucleotides. Oligonucleotides containing pyrimidine nucleotides with a propynyl group at the C5 position form more stable duplexes and triplexes than their corresponding pyrimidine derivatives. Purine with the same propyne substituent at the 7-position form even more stable duplexes and are hence preferred. Thus, in certain preferred embodiments, efficiency was further increased through the use of 7-propynyl purine nucleotides ( 7-propynyl derivatives of 8-aza-7-deaza-2'-deoxyguanosine and 8-aza-7-deaza-2'-deoxyadenine) which enhance binding affinity to an even greater degree than C5-propyne pyrimidine nucleotides. Such nucleotides are disclosed inter alia in He & Seela, 2002 Nucleic Acids Res. 30: 5485-5496.

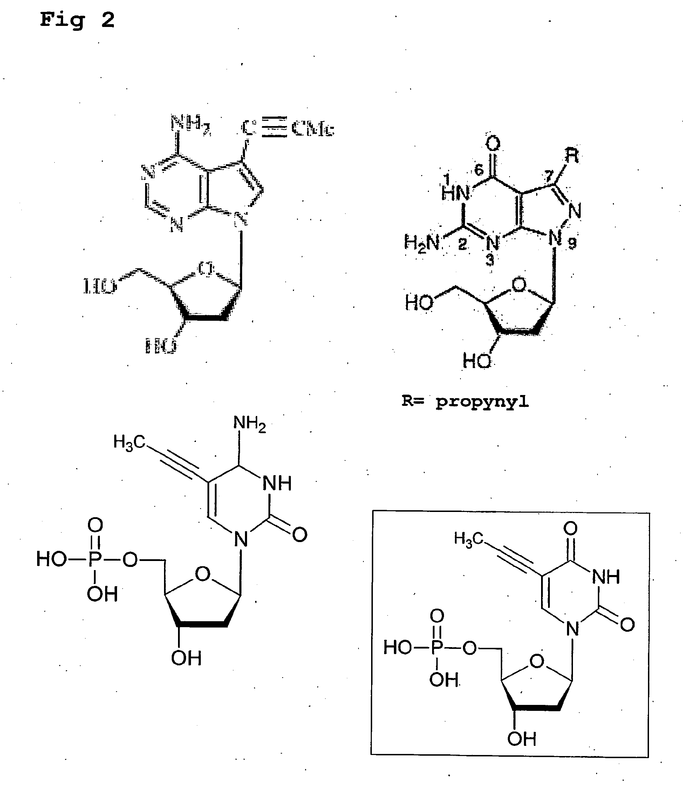

A propynyl group is a three carbon chain with a triple bond. The triple bond is covalently bound to the nucleotide basicstructure which is located at the $C^5$ position of the pyrimidine and at the 7-postion of the purine nucleotide (Fig. 2). Both cytosine and thymidine can be equipped with C5-propynyl group, resulting in $C^5$-propynyl-cytosine and $C^5$-propynyl-thymidine, respectively. A single $C^5$-propynyl-cytosine residue increases the $T_m$ by 2.8°C, a single $C^5$-propynyl-thymidine by 1.7°C.

(Froehler et al. 1993 Tetrahedron Letters 34: 1003-6; Lacroix et al. 1999 Biochemistry 38: 1893-1901; Ahmadian et al. 1998 Nucleic Acids Res. 26: 3127-3135; Colocci et al. 1994 J. Am.Chem.Soc 116: 785-786). This is attributed to the hydrophobic nature of 1-propyne groups at the C5 position and it also allows better stacking of the bases since the propyne group is planar with respect to the heterocyclic base.

The improved binding properties of oligonucleotides containing C5-propyne substituted pyrimidine groups has been exploited to alter a cellular process. An antisense oligonucleotide containing C5-propyne groups forms a more stable duplex with its target mRNA, leading to an increase in the inhibition of gene expression (Wagner et al. 1993 Science 260: 1510-1513; Flanagan et al. 1996 Nature Biotech. 14: 1139-1145; Meunier et al. 2001 Antisense & Nucleic Acid Drug Dev. 11: 117-123). Furthermore, these experiments demonstrate that such oligonucleotides are biologically active and that they can be tolerated by the cell. In the art on TNE, C5-propyne pyrimidine modification has been listed amongst a list of possible oligonucleotide modifications as alternatives for the chimeric molecules used in TNE. However, there is no indication in the art thus far that suggests that C5 propyne modified single-stranded DNA oligonucleotides enhances TNE efficiency significantly to the extent that has presently been found.

The delivery of the oligonucleotide can be achieved via electroporation or other conventional techniques that are capable of delivering either to the nucleus or the cytoplasm. In vitro testing of the method of the present disclosure can be achieved using the Cell Free system as is described i.a. in WO01/87914, WO03/027265, WO99/58702, WO01/92512.

As used herein, the capability of the donor oligonucleotide to influence the TNE depends on the type, location and amount of modified nucleotides that are incorporated in the donor oligonucleotide. This capability can be quantified for instance by normalising the binding affinity (or the binding energy (Gibbs Free Energy)) between conventional nucleotides at 1, i.e. for both AT and GC bindings, the binding affinity is normalised at 1. For the oligonucleotides of the present invention the Relative Binding Affinity (RBA) of each modified nucleotide is > 1. This is exemplified in a formula below:

$$RBA = \sum_{n} RBA(modified) - \sum_{m} RBA(unmodified) > 0$$

Wherein RBA is the total relative binding affinity, RBA(modified) is the sum of the relative binding affinity of the modified oligonucleotide with a length of n nucleotides and RBA(unmodified) is the sum of the relative binding affinity of the unmodified oligonucleotide with a length of m nucleotides. For example, an 100 bp oligonucleotide contains 10 modifications, each with a relative binding affinity of 1.1. The total RBA then equals: RBA = [(10*1.1) + (90*1.0)] - (100*1.0) = 1.

Note that the definition of RBA is in.principle independent of the length of the nucleotide strand that is compared. However, when RBAs of different strands are compared it is preferred that the strands have about the same length or that sections of comparable length are taken. Note that RBA does not take into account that modification can be grouped together on a strand. A higher degree of modification of a certain strand A compared to a strand B thus means that RBA (A) > RBA(B). For upstream and downstream sections, corresponding RBA values may be defined and used. To accommodate.the effect of the position of the modified nucleotide a weighing factor can be introduced into the RBA value. For instance, the effect of a modified nucleotide on the donor oligonucleotide adjacent to the mismatch can be larger than that of a modified nucleotide that is located at a distance five nucleotides removed from the mismatch. In the context of the present disclosure, RBA (Donor)> RBA (Acceptor).

In certain embodiments, the RBA value of the Donor may be at least 0.1 larger than the RBA of the Acceptor. In certain embodiments, the RBA value of the Donor may be at least 0.2, 0.3, 0.4, 0.5, 0.6, 0.7, 0.8, 0.9, 1.0, 1.5, 2.0, 2.5 larger than the RBA of the Acceptor. RBA values can be derived from conventional analysis of the modified binding affinity of the nucleotide, such as by molecular modelling, thermodynamic measurements etc. Alternatively they can be determined by measurement of Tm differences between modified and unmodified strands. Alternatively, the RBA can be expressed as the difference in Tm between the unmodified and the modified strand, either by measurement or by calculation using conventional formulates for calculating the Tm of a set of nucleotides, or by a combination of calculation and measurements.

The donor oligonucleotides according to the disclosure may contain further modifications to improve the hybridisation characteristics such that the donor exhibits increased affinity for the target DNA strand so that intercalation of the donor is easier. The donor oligonucleotide can also be further modified to become more resistant against nucleases, to stabilise the triplex or quadruplex structure. Modification of the C5 propyne substituted pyrimidine donor oligonucleotides can comprise phosphorothioate modification, 2-OMe substitutions, the use of LNAs (Locked nucleic acids), PNAs (Peptide nucleic acids), ribonucleotide and other bases that modifies, preferably enhances, the stability of the hybrid between the oligonucleotide and the acceptor stand.

Particularly useful among such modifications are PNAs, which are oligonucleotide analogues where the deoxyribose backbone of the oligonucleotide is replaced by a peptide backbone. One such peptide backbone is constructed of repeating units of N- (2-aminoethyl) glycine linked through amide bonds. Each subunit of the peptide backbone is attached to a nucleobase (also designated "base"), which may be a naturally occurring, non-naturally occurring or modified base. PNA oligomers bind sequence specifically to complementary DNA or RNA with higher affinity than either DNA or RNA. Accordingly, the resulting PNA/DNA or PNA/RNA duplexes have higher melting temperatures (Tm). In addition, the Tm of the PNA/DNA or PNA/RNA duplexes is much less sensitive to salt concentration than DNA/DNA or DNA/RNA duplexes. The polyamide backbone of PNAs is also more resistant to enzymatic degradation. The synthesis of PNAs is described, for example, in WO 92/20702 and WO 92/20703. Other PNAs are illustrated, for example, in WO93/12129 and United States Patent No. 5,539,082, issued July 23,1996. In addition, many scientific publications describe the synthesis of PNAs as well as their properties and uses. See, for example, Patel, Nature, 1993, 365, 490 ; Nielsen et al., Science, 1991, 254, 1497; Egholm, J. Am. Chem. Soc., 1992,114,1895; Knudson et al., Nucleic Acids Research, 1996,24,494; Nielsen et al., J. Am. Chem. Soc., 1996,118,2287 ; Egholm et al., Science, 1991,254,1497; Egholm et al., J. Am. Chem. Soc., 1992,114,1895; and Egholm et al., J Am. Chem. Soc., 1992,114,9677.

Useful further modifications of the C5-propyne substituted pyrimidine oligonucleotides of the present disclosure are also know as Super A and Super T , obtainable from Epoch Biosciences Germany. These modified nucleotides contain an additional substituent that sticks into the major groove of the DNA where it is believed to improve base stacking in the DNA duplex. See also Fig 4

Useful further modifications also include one or more monomers from the class of synthetic molecules known as locked nucleic acids (LNAs). LNAs are bicyclic and tricyclic nucleoside and nucleotide analogues and the oligonucleotides that contain such analogues. The basic structural and functional characteristics of LNAs and related analogues are disclosed in various publications and patents, including WO 99/14226, WO 00/56748, WO00/66604, WO 98/39352, United States Patent No. 6, 043, 060, and United States Patent No. 6,268,490.

The donor oligonucleotides of the disclosure can also be made chimeric, i.e. contain sections of DNA, RNA, LNA, PNA or combinations thereof.

Thus, in certain embodiments, the oligonucleotide of the invention further contains other, optionally non-methylated, modified nucleotides.

In certain embodiments, the oligonucleotide is resistant against nucleases. This is advantageous to prevent the oligonucleotide from being degraded by nucleases and enlarges the chance that the donor oligonucleotide can find its target (acceptor molecule).

In certain embodiments of the disclosure, the nucleotide in the oligonucleotide at the position of the mismatch can be modified. Whether or not the mismatch can be modified will depend to a large extent on the exact mechanism of the targeted nucleotide exchange or of the cell's DNA repair mechanism using the difference in affinity between the donor and acceptor strands. The same holds for the exact location of the other modified positions in the neighbourhood or vicinity of the mismatch. However, based on the disclosure presented herein, such an oligonucleotide can be readily designed and tested, taking into account the test procedures for suitable oligonucleotides as described herein elsewhere. In certain embodiments, the nucleotide at the position of the mismatch is not modified. In certain embodiments, modification is adjacent to the mismatch, preferably within 2, 3, 4, 5, 6 or 7 nucleotides of the mismatch. In certain embodiments, modification is located at a position downstream from the mismatch. In certain embodiments, modification is located at a position upstream from the mismatch. In certain embodiments, the modification is located from 10 bp to 10kB from the mismatch, preferably from 50 to 5000 bp, more preferably from 100 to 500 from the mismatch.

The oligonucleotides that are used as donors can vary in length but generally vary in length between 10 and 500 nucleotides, with a preference for 11 to 100 nucleotides, preferably from 15 to 90, more preferably from 20 to 70 most preferably from 30 to 60 nucleotides.

In one aspect, the disclosure pertains to a method for the targeted alteration of a duplex acceptor DNA sequence, comprising combining the duplex acceptor DNA sequence with a donor oligonucleotide, wherein the duplex acceptor DNA sequence contains a first DNA sequence and a second DNA sequence which is the complement of the first DNA sequence and wherein the donor oligonucleotide comprises a domain that comprises at least one mismatch with respect to the duplex acceptor DNA sequence to be altered, preferably with respect to the first DNA sequence, and wherein a section of the donor oligonucleotide is modified with C5-propynyl substituted pyrimidines to express a higher degree of affinity to the first DNA sequence compared to an unmodified nucleotide at that position in the oligonucleotide and/or wherein the second DNA is methylated to a lower degree of methylation than the corresponding section of the donor oligonucleotide, in the presence of proteins that are capable of targeted nucleotide exchange.

The disclosure is, in its broadest form, generically applicable to all sorts of organisms such as humans, animals, plants, fish, reptiles, insects, fungi, bacteria and so on. The disclosure is applicable for the modification of any type of DNA, such as DNA derived from genomic DNA, linear DNA, artificial chromosomes, nuclear chromosomal DNA, organelle chromosomal DNA, BACs, YACs. The disclosure can be performed *in vivo* as well as *ex vivo.*

The disclosure is, in its broadest form, applicable for many purposes for altering a cell, correcting a mutation by restoration to wild type, inducing a mutation, inactivating an enzyme by disruption of coding region, modifying bioactivity of an enzyme.by altering coding region, modifying a protein by disrupting the coding region.

The disclosure also relates to the use of oligonucleotides essentially as described hereinbefore, for altering a cell, correcting a mutation by restoration to wild type, inducing a mutation, inactivating an enzyme by disruption of coding region, modifying bioactivity of an enzyme by altering coding region, modifying a protein by disrupting the coding region, mismatch repair, targeted alteration of (plant)genetic material, including gene mutation, targeted gene repair and gene knockout

The disclosure further relates to kits, comprising one or more oligonucleotides as defined herein elsewhere, optionally in combination with proteins that are capable of inducing MRM, and in particular that are capable of TNE.

The disclosure further relates to modified genetic material obtained by the method of the present disclosure, to cells and organisms that comprise the modified genetic material, to plants or plant parts that are so obtained.

The disclosure relates in particular to the use of the TNE method using the propyne-modified oligonucleotides of the invention to provide for herbicide resistance in plants. In particular the disclosure relates to plants that have been provided with resistance against herbicides, in particular kanamycin, glyphosate and/or carbenicillin.

Description of the Figures

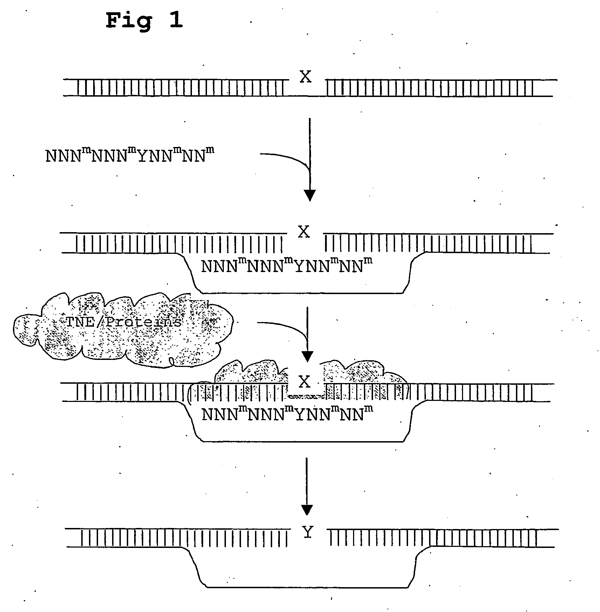

Figure 1: Schematic representation of targeted nucleotide exchange. An acceptor duplex DNA strand containing a nucleotide that is to be exchanged (X) is brought into contact with a C5-propyne pyrimidine modified donor oligonucleotide (schematically given as NNN^mNNN^mYNN^mNN^m) containing the nucleotide to be inserted (Y). The triplex structure is subjected to or brought into contact with an environment that is capable of TNE or at least with proteins that are capable of performing TNE, such as are known as the cell-free enzyme, mixture or a cell-free extract (see i.a. WO99/58702, WO01/73002).

**Figure 2:** Chemical structures of 5-propynyl-deoxythymidine, 5-propynyl-deoxycytosine, 2'-Deoxy-7-propynyl-7-deaza-adenosine and the 2'deoxy-7-propynyl-deaza-guanosine.

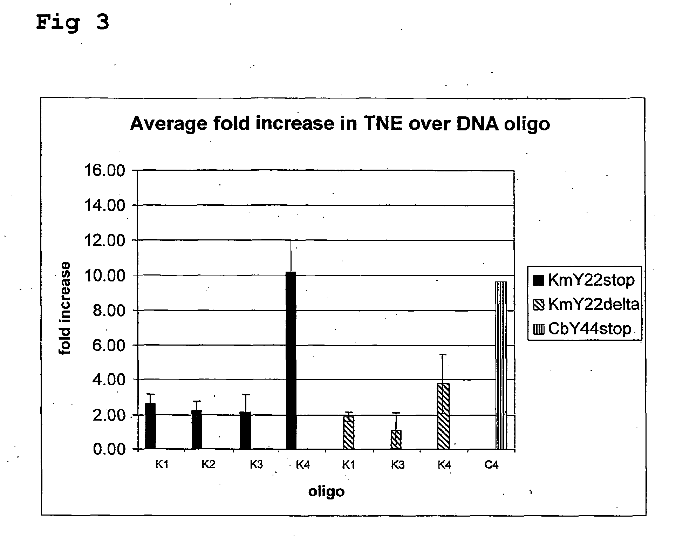

**Figure 3:** The TNE repair efficiency of oligonucleotides containing C5-propyne pyrimidine nucleotides as measured using the cell free assay. Per experiment the repair efficiency using the normal DNA oligonucleotide was determined and set at a value of 1. The fold increase indicates the increase in repair seen using the C5-propyne pyrimidine containing oligonucleotides compared to the repair efficiency obtained when using the normal DNA oligonucleotide. Similar results were obtained using propynylated purines.

Example

**Materials and Methods**

Oligonucleotides containing C5-propyne pyrimidines were purchased from Trilink Biotech or GeneLink. The sequences of the oligos used are shown below. The plasmid used in the experiments was a derivative of pCR2.1 (Invitrogen) that contains genes conferring both kanamycin and carbenicillin resistance. In frame stop codons and deletions were introduced into the kanamycin and carbenicillin as previously described (Sawano et al. 2000 Nucleic Acids Res. 28: e78). Plasmid KmY22stop has a TAT to TAG mutation at codon Y22 in the kanamycin ORF while plasmid CbY44stop has a TAC to TAG conversion at codon Y44 in the carbenicillin ORF. In plasmid KmY22. the third nucleotide of the Y22 codon (TA<u>T</u>) was deleted giving a frame shift.

Defective kanamycin and carbenicillin genes and the oligonucleotides used in the cell free system:

```
Km WT        GAG AGG CTA TTC GGC TAT GAC TGG GCA CAA CAG
              E   R   L   F   G   Y   D   W

KmY22stop GAG AGG CTA TTC GGC TAG GAC TGG GCA CAA CAG
              E   R   L   F   G   *

KmY22Δ       GAG AGG CTA TTC GGC TA_ GAC TGG GCA CAA CAG
              E   R   L   F   G   *

Cb WT     GGT GCA CGA GTG GGT TAC ATC GAA CTG GAT CTC
              G   A   R   V   G   Y   I   E   L   D   L

CbY44stop GGT GCA CGA GTG GGT TAG ATC GAA CTA GAT CTC
              G   A   R   V   G   *
```

| Oligo | Sequence | % propynyl groups | SEQ ID |
|---|---|---|---|
| K1 | tgtgcccagty**gz**agccgaatagc | 8 | 1 |
| K2 | tgtgyyyagty**g**tagyygaatagy | 29 | 2 |
| K3 | **zgz**gcccagzcgzagccgaazagc | 20 | 3 |
| K4 | zgzgyyyag**zygz**agyygaa**z**agy | 50 | 4 |
| C4 | aygag**z**gggg**zz**ata**z**ygaay**z**gga | 33 | 5 |

The relevant sequence of the kanamycin and carbenicillin open reading frames and the amino acids encoded are shown. The single nucleotide mutations producing a stop codon (TAG, *) were introduced as previously described (Sawano et al. 2000 Nucleic Acids Res. 28: e78). The sequences of the oligonucleotides used in the experiments are shown. Oligonucleotides K1-K4 were used to repair the KmY22stop and KmY22Δ mutation to an alternative tyrosine encoding codon (TAC). Similarly, the oligonucleotide C1 was used to change the CbY44 mutation (TAG) to the.alternative tyrosine codon (TAT). The mismatch nucleotide in each oligonucleotide is underlined. The propyne derivative nucleotides are indicated (Y=5-propynyl-deoxycytidine, Z=5-propynyl-deoxyuracil). The oligonucleotide binding regions are underlined on the kanamycin and carbenicillin ORF's. Oligonucleotides K1-K4 are complementary to the kanamycin coding sequence whereas C1 is complementary to the carbenicillin non-coding strand.

Cell free assays were performed as follows. Flower buds from *Arabidopsis thaliana* (ecotype Col-0) were collected and ground under nitrogen. 200µl protein isolation buffer (20mM HEPES pH7.5, 5mM KC1, 1.5mM $MgCl_2$, 10mM DTT, 10% (v/v) glycerol, 1% (w/v) PVP) was added. The plant debris was pelleted by centrifugation at 14k RPM for 30 mins and the supernatant was stored at -80°C. The protein concentration was measured using the NanoOrange Kit (Molecular Probes, Inc). A typical isolation resulted in a protein concentration of approximately 3-4µg/µl. The cell free reactions contained the following components. 1µg plasmid DNA (KmY22stop or CbY44stop), 100ng of oligonucleotide, 30µg total plant protein, 4µl sheared salmon sperm DNA (3µg/µl), 2µl protease inhibitor mix (50x conc: Complete EDTA-free protease inhibitor cocktail tablets, Roche Diagnostics), 50µl 2x cell free reaction buffer (400mM Tris pH7.5, 200mM $MgCl_2$, 2mM DTT, 0.4mM spermidine, 50mM ATP, 2mM each CTP, GTP, UTP, 0.1mm each dNTPs and 10mM NAD) made up to a total volume of 100µl with water. The mixture was incubated at 37°C for 1 hr. The plasmid DNA was then isolated as follows. 100µl $H_2O$ was added to each reaction to increase the volume followed by 200µl alkaline buffered phenol (pH8-10). This was vortexed briefly and then centrifuged and 13k rpm for 3 mins. The upper aqueous phase was transferred to a new tube and 200µl chloroform was then added. This was vortexed briefly, spun at 13k rpm for 3 mins and the aqueous phase transferred to a new tube. The DNA was precipitated by addition of 0.7 volume 2-propanol and the pellet resuspended in TE. To eliminate any co-purified oligonucleotide the DNA was passed over a Qiagen PCR purification column and the plasmid DNA eluted in a final volume of 30µl. 2µl of plasmid DNA was electroporated to 18µl of DH10B (Invitrogen) electrocompetent cells. After electroporation the cells were allowed to recover in SOC medium for 1hr at 37°C. After this period, for the experiments using KmY22stop and KmY22., kanamycin was added to a concentration of 100µg/ml and the cells were incubated for a further 3 hours. For the experiments using CbY44stop, the cells were plated out on solid medium directly after the recovery period. Solid media contained 100µg/ml kanamycin or carbenicillin. For the KmY22stop and KmY22. experiments, the number of TNE events were detected on kanamycin medium and the electroporation efficiency was calculated by counting the number of colonies obtained from a $10^{-4}$ and $10^{-5}$ dilution of the electroporation plated out on carbenicillin medium. For experiments done using CbY44stop, these selection conditions were reversed. The TNE efficiency was calculated by dividing the number of TNE events by the total number of transformed cells.

**Results**

The oligonucleotides were designed to produce a single nucleotide substitution at the stop codon (TAG) introduced into the kanamycin or carbenicillin ORFs of KmY22stop and CbY44stop so that the codon again codes for the correct amino acid. In plasmid KmY22. the oligonucleotides were designed to add a single nucleotide, thus restoring the ORF. To establish the optimal number of C5-propyne pyrimidines, a series of oligonucleotides was made. Oligo K1 contains two C5-propyne pyrimidines flanking the mismatch nucleotide. In oligo K2, all the cytosine nucleotides are replaced by '5-propynyl-deoxycytidine. In oligo K3, all the thymidine nucleotides are replaced by 5-propynyl-deoxyuracil. In oligos K4 and C4 all the pyrimidines are replaced by C5-propyne pyrimidines. In each experiment unmodified DNA oligonucleotides and C5-propyne modified oligonucleotides were run in parallel. In each experiment, the TNE efficiency obtained using the normal DNA oligonucleotide was arbitrarily set at 1 and the TNE efficiency of the C5-propyne modified oligonucleotides was subsequently expressed as the fold increase over the normal DNA oligonucleotide. As shown in figure 3, C5-propyne modified oligonucleotides worked more efficiently than normal DNA in our assay. The efficiency of both substitutions and insertions was increased. The overall repair efficiency of substitutions was higher than that for the insertions. The enhancement that is observed is dependent on the percentage of C5-propyne nucleotides present. Oligonucleotides (24mers) containing 2 (K1), 7 (K2) or 5 (K3) C5-propyne pyrimidines all show a 2-3 fold increase-over DNA oligonucleotides. However, the greatest increase in repair efficiency (6-10 fold) is observed using oligonucleotides where all 12 pyrimidine nucleotides are replaced by C5-propyne pyrimidines (K4 & C4). It is important to note that this effect is not only limited to repair at KmY22 as the repair of the stop codon at CbY44 is also enhanced when C5-propyne modified oligonucleotides are used.

Plasmids were purified from colonies in which we assumed that conversion of the stop codon had occurred and the antibiotic resistance genes were subsequently sequenced to confirm that the stop codons had indeed been altered.

Thus it was found that oligonucleotides containing C5-propyne pyrimidine nucleotides have improved antisense properties. It was demonstrated that, using an *in vitro* TNE assay, the cell free system, oligonucleotides containing C5-propyne pyrimidine nucleotides do show significantly higher levels of TNE compared to the TNE efficiency obtained using normal DNA oligonucleotides. This enhancement can be as high as 10 fold, and is independent of the oligonucleotide sequence or the locus to be altered. This enhancement was improved further by targeting pyrimidine rich sequences so that percentage of C5-propyne pyrimidine nucleotides is maximised. Efficiency was also as well as further increased through the use of 7-propynyl purine nucleotides which enhance binding affinity to an even greater degree than C5-propyne pyrimidine nucleotides (He & Seela, 2002 Nucleic Acids Res. 30: 5485-5496). Combining propyne purines and pyrimidines allows the production of oligonucleotides in which all the nucleotides carry propynyl groups on the bases.

**Claims**

1. Method for targeted alteration of a duplex acceptor DNA sequence, comprising combining the duplex acceptor DNA sequence with a donor oligonucleotide, wherein the duplex acceptor DNA sequence contains a first DNA sequence and a second DNA sequence which is the complement of the first DNA sequence and wherein the donor oligonucleotide comprises a domain that comprises at least one mismatch with respect to the duplex acceptor DNA sequence to be altered, preferably with respect to the first DNA sequence, and wherein the oligonucleotide comprises at least one section that contains at least one modified nucleotide having a higher binding affinity compared to naturally occurring A, C, T or G, and wherein the modified nucleotide binds stronger to a nucleotide in an opposite position in the first DNA sequence as compared to a naturally occurring nucleotide complementary to the nucleotide in an opposite position in the first DNA sequence, in the presence of proteins that are capable of targeted nucleotide exchange, wherein the modified nucleotide is a C7-propyne purine or C5-propyne pyrimidine, wherein in the oligonucleotide at least 50% of the pyrimidines and purines are replaced by their propynylated derivatives, and wherein said method is not for the treatment of the human body or animal body by surgery or therapy, and wherein said method is not for targeted alteration of DNA in humans.

2. Method according to claim 1, wherein in the oligonucleotide at least 75% of the pyrimidines and purines are replaced by their propynylated derivatives, preferably at least 90%.

3. Method according to claims 1 - 2 wherein the purine is adenosine or guanosine and/or the pyrimidine is cytosine, uracil or thymidine.

4. Method according to claims 1 -3 wherein modified nucleotide is a pyrimidine.

5. Method according to any of claims 1-3 wherein modified nucleotide is a purine.

6. Method according to anyone of the previous claims, wherein the nucleotide at the position of the mismatch is not modified.

7. Method according to anyone of the previous claims, the oligonucleotide having a length from 10 to 500 nucleotides.

8. Method according to claim 1, wherein the alteration is within a cell preferably selected from the group consisting of a plant cell, a fungal cell, a rodent cell, a primate cell, a human cell or a yeast cell.

9. Method according to claim 1, wherein the proteins are derived from a cell extract.

10. Method according to claim 9, wherein the cell extract is selected from the group consisting of a plant cell extract, a fungal cell extract, a rodent cell extract, a primate cell extract, a human cell extract or a yeast cell extract.

11. Method according to claim 1, wherein the alteration is a deletion, a substitution or an insertion of at least one nucleotide.

12. Method according to any of the previous claims, wherein the cell is a eukaryotic cell, a plant cell, a non-human mammalian cell or a human cell.

13. Method according to any of the previous claims, wherein the target DNA is from fungi, bacteria, plants, mammals or humans.

14. Method according to any of the previous claims, wherein the duplex DNA is from genomic DNA, linear DNA, mammalian artificial chromosomes, bacterial artificial chromosomes, yeast artificial chromosomes, plant artificial chromosomes, nuclear chromosomal DNA, organelle chromosomal DNA, or episomal DNA.

15. Method according to any of the previous claims, for altering a cell, correcting a mutation by restoration to wild type, inducing a mutation, inactivating an enzyme by disruption of coding region, modifying bioactivity of an enzyme by altering coding region, modifying a protein by disrupting the coding region.

16. Method according to any of the previous claims, wherein the method is performed ex vivo.

17. Use of an oligonucleotide as described in any of the claims 1 - 16 in targeted alteration of a duplex acceptor DNA sequence, wherein the use is ex vivo if the organism is human or animal.

**Patentansprüche**

1. Verfahren zur zielgerichteten Änderung einer Duplex-Akzeptor-DNA-Sequenz, umfassend das Kombinieren der Duplex-Akzeptor-DNA-Sequenz mit einem Donor-Oligonukleotid, wobei die Duplex-Akzeptor-DNA-Sequenz eine erste DNA-Sequenz und eine zweite DNA-Sequenz, welche das Komplement der ersten DNA-Sequenz darstellt, enthält, und wobei das Donor-Oligonukleotid eine Domäne umfasst, die wenigstens eine Fehlpaarung bezüglich der zu ändernden Duplex-Akzeptor-DNA-Sequenz aufweist, vorzugsweise bezüglich der ersten DNA-Sequenz, und wobei das Oligonukleotid wenigstens einen Abschnitt umfasst, der wenigstens ein modifiziertes Nukleotid enthält, welches eine höhere Bindungsaffinität verglichen mit natürlicherweise vorkommenden A, C, T oder G aufweist, und wobei das modifizierte Nukleotid stärker an ein Nukleotid einer gegenüberliegenden Position in der ersten DNA-Sequenz bindet, verglichen mit einem natürlicherweise vorkommenden, zu dem Nukleotid komplementären Nukleotid in einer gegenüberliegenden Position in der ersten DNA-Sequenz, in Anwesenheit von Proteinen, die zu einem zielgerichteten Nukleotidaustausch in der Lage sind, wobei das modifizierte Nukleotid ein C7-Propin-Purin oder C5-Propin-Pyrimidin ist, wobei in dem Oligonukleotid wenigstens 50% der Pyrimidine und Purine durch ihre propinylierten Derivate ersetzt sind, und wobei das besagte Verfahren nicht zur Behandlung des menschlichen oder tierischen Körpers durch Chirurgie oder Therapie ist, und wobei das besagte Verfahren nicht für eine zielgerichtete Änderung von DNA in Menschen ist.

2.  Verfahren gemäß Anspruch 1, wobei in dem Oligonukleotid wenigstens 75% der Pyrimidine und Purine durch ihre propinylierten Derivate ersetzt sind, vorzugsweise wenigstens 90%.

3.  Verfahren gemäß Anspruch 1 - 2 wobei das Purin Adenosin oder Guanosin ist und/oder das Pyrimidin Cytosin, Uracil oder Thymidin ist.

4.  Verfahren gemäß Ansprüchen 1 - 3, wobei das modifizierte Nukleotid ein Pyrimidin ist.

5.  Verfahren gemäß Ansprüchen 1 - 3, wobei das modifizierte Nukleotid ein Purin ist.

6.  Verfahren gemäß einem beliebigen der vorangegangenen Ansprüche, wobei das Nukleotid an der Position der Fehlpaarung nicht modifiziert ist.

7.  Verfahren gemäß einem beliebigen der vorangegangenen Ansprüche, wobei das Oligonukleotid eine Länge von 10 bis 500 Nukleotiden aufweist.

8.  Verfahren gemäß Anspruch 1, wobei die Änderung innerhalb einer Zelle ist, vorzugsweise ausgewählt aus der Gruppe bestehend aus einer pflanzlichen Zelle, einer Pilzzelle, einer Nagetierzelle, einer Primatenzelle, einer menschlichen Zelle, oder einer Hefezelle.

9.  Verfahren gemäß Anspruch 1, wobei die Proteine von einem Zellextrakt abgeleitet sind.

10. Verfahren gemäß Anspruch 9, wobei der Zellextrakt ausgewählt ist aus der Gruppe bestehend aus, einem pflanzlichen Zellextrakt, einem Pilzzellextrakt, einem Nagetierzellextrakt, einem Primatenzellextrakt, einem menschlichen Zellextrakt, oder einem Hefezellextrakt.

11. Verfahren gemäß Anspruch 1, wobei die Änderung eine Deletion, eine Substitution, oder eine Insertion von wenigstens einem Nukleotid ist.

12. Verfahren gemäß einem beliebigen der vorangegangenen Ansprüche, wobei die Zelle eine eukaryontische Zelle, eine Pflanzenzelle, eine nicht-menschliche Säugerzelle, oder eine humane Zelle ist.

13. Verfahren gemäß einem beliebigen der vorangegangenen Ansprüche, wobei die Ziel-DNA aus Pilzen, Bakterien, Pflanzen, Säugern oder Menschen ist.

14. Verfahren gemäß einem beliebigen der vorangegangenen Ansprüche, wobei die Duplex-DNA aus genomischer DNA, linearer DNA, artifiziellen Säugerchromosomen, artifiziellen Bakterienchromosomen, artifiziellen Hefechromosomen, artifiziellen Pflanzenchromosomen, nukleärer chromosomaler DNA, Organell-chromosomaler DNA, oder episomaler DNA ist.

15. Verfahren gemäß einem beliebigen der vorangegangenen Ansprüche, zum Ändern einer Zelle, zum Korrigieren einer Mutation durch Wiederherstellen des Wildtyps, zum Einbringen einer Mutation, zum Inaktivieren eines Enzyms durch Unterbrechung der kodierenden Region, zum Modifizieren der Bioaktivität eines Enzyms durch Ändern der kodierenden Region, zum Modifizieren eines Proteins durch Unterbrechen der kodierenden Region.

16. Verfahren gemäß einem beliebigen der vorangegangenen Ansprüche, wobei das Verfahren ex vivo ausgeführt wird.

17. Verwendung eines Oligonukleotids, wie in einem beliebigen der Ansprüche 1-16 beschrieben, in der zielgerichteten Änderung einer Duplex-Akzeptor-DNA-Sequenz, wobei die Verwendung ex vivo stattfindet, falls der Organismus Mensch oder Tier ist.

**Revendications**

1. Procédé de modification ciblée d'une séquence ADN accepteur duplex, comprenant la combinaison de la séquence d'ADN accepteur duplex avec un oligonucléotide donneur, dans lequel la séquence d'ADN accepteur duplex contient une première séquence d'ADN et une deuxième séquence d'ADN qui est le complément de la première séquence d'ADN et dans lequel l'oligonucléotide donneur comprend un domaine qui comprend au moins un mésappariement par rapport à la séquence d'ADN accepteur duplex à modifier, de préférence par rapport à la première séquence d'ADN, et dans lequel l'oligonucléotide comprend au moins une section qui contient au moins un nucléotide modifié ayant une affinité de liaison plus élevée par rapport à A, C, T ou G, d'origine naturelle, et dans lequel le nucléotide modifié se lie plus fortement à un nucléotide dans une position opposée dans la première séquence d'ADN par rapport à un nucléotide d'origine naturelle complémentaire du nucléotide dans une position opposée dans la première séquence d'ADN, en présence de protéines qui sont capables d'échange de nucléotide ciblé, dans lequel le nucléotide modifié est une C7-propyne purine ou une C5-propyne pyrimidine, dans lequel, dans l'oligonucléotide au moins 50% des pyrimidines et des purines sont remplacées par leurs dérivés propynylés, et dans lequel ledit procédé n'est pas destiné au traitement du corps humain ou du corps animal par chirurgie ou thérapie, et dans lequel ledit procédé n'est pas destiné à une modification ciblée de l'ADN chez les humains.

2. Procédé selon la revendication 1, dans lequel, dans l'oligonucléotide au moins 75% des pyrimidines et des purines sont remplacées par leurs dérivés propynylés, de préférence au moins 90%.

3. Procédé selon les revendications 1 à 2, dans lequel la purine est l'adénosine ou la guanosine et/ou la pyrimidine est la cytosine, l'uracile ou la thymidine.

4. Procédé selon les revendications 1 à 3, dans lequel le nucléotide modifié est une pyrimidine.

5. Procédé selon l'une quelconque des revendications 1 à 3, dans lequel le nucléotide modifié est une purine.

6. Procédé selon l'une quelconque des revendications précédentes, dans lequel le nucléotide à la position du mésappariement n'est pas modifié.

7. Procédé selon l'une quelconque des revendications précédentes, l'oligonucléotide ayant une longueur de 10 à 500 nucléotides.

8. Procédé selon la revendication 1, dans lequel la modification se situe au sein d'une cellule choisie de préférence dans le groupe constitué par une cellule végétale, une cellule fongique, une cellule de rongeur, une cellule de primate, une cellule humaine ou une cellule de levure.

9. Procédé selon la revendication 1, dans lequel les protéines sont dérivées d'un extrait cellulaire.

10. Procédé selon la revendication 9, dans lequel l'extrait cellulaire est choisi dans le groupe constitué par un extrait de cellules végétales, un extrait de cellules fongiques, un extrait de cellules de rongeur, un extrait de cellules de primate, un extrait de cellules humaines ou un extrait de cellules de levure.

11. Procédé selon la revendication 1, dans lequel la modification est une délétion, une substitution ou une insertion d'au moins un nucléotide.

12. Procédé selon l'une quelconque des revendications précédentes, dans lequel la cellule est une cellule eucaryote, une cellule végétale, une cellule de mammifère non humain ou une cellule humaine.

13. Procédé selon l'une quelconque des revendications précédentes, dans lequel l'ADN cible provient de champignons, de bactéries, de plantes, de mammifères ou d'humains.

14. Procédé selon l'une quelconque des revendications précédentes, dans lequel l'ADN duplex provient d'un ADN génomique, d'un ADN linéaire, de chromosomes artificiels de mammifère, de chromosomes artificiels bactériens, de chromosomes artificiels de levure, de chromosomes artificiels de plantes, d'un ADN chromosomique nucléaire, d'un ADN chromosomique d'organite, ou d'un ADN épisomal.

15. Procédé selon l'une quelconque des revendications précédentes, destiné à modifier une cellule, corriger une mutation par la restauration du type sauvage, induire une mutation, inactiver une enzyme par la destruction de la région codante, modifier la bioactivité d'une enzyme par l'altération de la région codante, modifier une protéine par la désorganisation de la région codante.

16. Procédé selon l'une quelconque des revendications précédentes, dans lequel le procédé est effectué *ex vivo.*

17. Utilisation d'un oligonucléotide selon l'une quelconque des revendications 1 à 16 dans la modification ciblée d'une séquence ADN accepteur duplex, dans laquelle l'utilisation s'effectue *ex vivo* si l'organisme est un humain ou un animal.

## Fig 1

# Fig 2

R= propynyl

# Fig 3

Average fold increase in TNE over DNA oligo

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0173002 A
- WO 03027265 A
- WO 0187914 A
- WO 9958702 A
- WO 9748714 A
- WO 0210364 A
- WO 0192512 A
- WO 9220702 A
- WO 9220703 A
- WO 9312129 A
- US 5539082 A
- WO 9914226 A
- WO 0056748 A
- WO 0066604 A
- WO 9839352 A
- US 6043060 A
- US 6268490 B
- NL 2005000884 W
- NL 2006000244 W

**Non-patent literature cited in the description**

- **ALEXEEV ; YOON.** *Nature Biotechnol.,* 1998, vol. 16, 1343
- **RICE.** *Nature Biotechnol.,* 2001, vol. 19, 321
- **KMIEC.** *J. Clin. Invest.,* 2003, vol. 112, 632
- **RICE et al.** *Nat.Biotech.,* vol. 19, 321-326
- **BEETHAM et al.** *Proc.Natl.Acad.Sci.USA,* 1999, vol. 96, 8774-8778
- **KOCHEVENKO et al.** *Plant Phys.,* 2003, vol. 132, 174-184
- **OKUZAKI et al.** *Plant Cell Rep.,* 2004, vol. 22, 509-512
- **LIU et al.** *Nuc.Acids Res.,* 2002, vol. 30, 2742-2750
- **PAREKH-OLMEDO et al.** *Gene Therapy,* 2005, vol. 12, 639-646
- **DONG et al.** *Plant Cell Rep.,* 2006, vol. 25, 457-65
- **COLE-STRAUSS et al.** *Nucleic Acids Res.,* 1999, vol. 27, 1323-1330
- **GAMPER et al.** *Nucleic Acids Res.,* 2000, vol. 28, 4332-4339
- **KMIEC et al.** *Plant J.,* 2001, vol. 27, 267-274
- **ZHU et al.** *Nature Biotech.,* 2000, vol. 18, 555-558
- **FEDIER ; FINK.** *Int. J Oncol. 2004,* 2004, vol. 24 (4), 1039-47
- **HE ; SEELA.** *Nucleic Acids Res.,* 2002, vol. 30, 5485-5496
- **FROEHLER et al.** *Tetrahedron Letters,* 1993, vol. 34, 1003-6
- **LACROIX et al.** *Biochemistry,* 1999, vol. 38, 1893-1901
- **AHMADIAN et al.** *Nucleic Acids Res.,* 1998, vol. 26, 3127-3135
- **COLOCCI et al.** *J. Am.Chem.Soc,* 1994, vol. 116, 785-786
- **WAGNER et al.** *Science,* 1993, vol. 260, 1510-1513
- **FLANAGAN et al.** *Nature Biotech.,* 1996, vol. 14, 1139-1145
- **MEUNIER et al.** *Antisense & Nucleic Acid Drug Dev.,* 2001, vol. 11, 117-123
- **PATEL.** *Nature,* 1993, vol. 365, 490
- **NIELSEN et al.** *Science,* 1991, vol. 254, 1497
- **EGHOLM.** *J. Am. Chem. Soc.,* 1992, vol. 114, 1895
- **KNUDSON et al.** *Nucleic Acids Research,* 1996, vol. 24, 494
- **NIELSEN et al.** *J. Am. Chem. Soc.,* 1996, vol. 118, 2287
- **EGHOLM et al.** *Science,* 1991, vol. 254, 1497
- **EGHOLM et al.** *J. Am. Chem. Soc.,* 1992, vol. 114, 1895
- **EGHOLM et al.** *J Am. Chem. Soc.,* 1992, vol. 114, 9677
- **SAWANO et al.** *Nucleic Acids Res.,* 2000, vol. 28, e78
- **SAWANO et al.** *Nucleic Acids Res.,* 2000, vol. 28, 78

SEQUENCE LISTING

<110> Keygene NV

<120> Improved targeted nucleotide exchange with propynyl modified oligonucleotides

<130> P27711PC02

<150> PCT/NL2005/000884
<151> 2005-12-22

<150> PCT/NL2006/000244
<151> 2006-05-09

<160> 5

<170> PatentIn version 3.3

<210> 1
<211> 24
<212> DNA
<213> Artificial

<220>
<223> modified oligonucleotide Y=5-propynyl-deoxycytidine, Z=5-propynyl-deoxyuracil

<220>
<221> modified_base
<222> (11)..(11)
<223> 5-propynyl-deoxycytidine

<220>
<221> modified_base
<222> (13)..(13)
<223> 5-propynyl-deoxyuracil

<400> 1
tgtgcccagt cgtagccgaa tagc          24

<210> 2
<211> 24
<212> DNA
<213> Artificial

<220>
<223> Modified oligonucleotide, Y=5-propynyl-deoxycytidine, Z=5-propynyl-deoxyuracil

<220>
<221> modified_base
<222> (5)..(7)
<223> 5-propynyl-deoxycytidine

<220>
<221> modified_base
<222> (11)..(11)
<223> 5-propynyl-deoxycytidine

<220>
<221> modified_base
<222> (16)..(17)
<223> 5-propynyl-deoxycytidine

<220>
<221> modified_base
<222> (24)..(24)
<223> 5-propynyl-deoxycytidine

<400> 2
tgtgcccagt cgtagccgaa tagc          24

<210> 3
<211> 24
<212> DNA
<213> Artificial

<220>
<223> Modified oligonucleotide, Z=5-propynyl-deoxyuracil

<220>
<221> modified_base
<222> (1)..(1)
<223> 5-propynyl-deoxyuracil

<220>
<221> modified_base
<222> (3)..(3)
<223> 5-propynyl-deoxyuracil

<220>
<221> modified_base
<222> (10)..(10)
<223> 5-propynyl-deoxyuracil

<220>
<221> modified_base
<222> (13)..(13)
<223> 5-propynyl-deoxyuracil

<220>
<221> modified_base
<222> (21)..(21)
<223> 5-propynyl-deoxyuracil

<400> 3
tgtgcccagt cgtagccgaa tagc          24

<210> 4
<211> 24
<212> DNA
<213> Artificial

<220>
<223> modified oligonucleotide

<220>
<221> modified_base
<222> (1)..(1)
<223> 5-propynyl-deoxyuracil

<220>
<221> modified_base
<222> (3)..(3)
<223> 5-propynyl-deoxyuracil

<220>
<221> modified_base
<222> (5)..(7)
<223> 5-propynyl-deoxycytidine

<220>
<221> modified_base
<222> (10)..(10)
<223> 5-propynyl-deoxyuracil

<220>
<221> modified_base
<222> (11)..(11)
<223> 5-propynyl-deoxycytidine

<220>
<221> modified_base
<222> (13)..(13)
<223> 5-propynyl-deoxyuracil

<220>
<221> modified_base
<222> (16)..(17)
<223> 5-propynyl-deoxycytidine

<220>
<221> modified_base
<222> (21)..(21)
<223> 5-propynyl-deoxyuracil

<220>
<221> modified_base
<222> (24)..(24)
<223> 5-propynyl-deoxycytidine

<400> 4
tgtgcccagt cgtagccgaa tagc          24

<210> 5
<211> 24

<212> DNA
<213> Artificial

<220>
<223> modified oligonucleotide

<220>
<221> modified_base
<222> (2)..(2)
<223> 5-propynyl-deoxycytidine

<220>
<221> modified_base
<222> (6)..(6)
<223> 5-propynyl-deoxyuracil

<220>
<221> modified_base
<222> (10) .. (10)
<223> 5-propynyl-deoxyuracil

<220>
<221> modified_base
<222> (11)..(11)
<223> 5-propynyl-deoxyuracil

<220>
<221> modified_base
<222> (16)..(16)
<223> 5-propynyl-deoxycytidine

<220>
<221> modified_base
<222> (20)..(20)
<223> 5-propynyl-deoxycytidine

<220>
<221> modified_base
<222> (21)..(21)
<223> 5-propynyl-deoxyuracil

<400> 5
acgagtgggt tatatcgaac tgga          24.